**Europäisches Patentamt**

**European Patent Office**

**Office européen des brevets**

(19)

(11) Numéro de publication : **0 472 462 A1**

(12)

# DEMANDE DE BREVET EUROPEEN

(21) Numéro de dépôt : **91402267.8**

(51) Int. Cl.$^5$ : **C07C 5/41,** C07C 2/00, C07C 15/00

(22) Date de dépôt : **19.08.91**

(30) Priorité : **24.08.90 FR 9010701**

(43) Date de publication de la demande :
**26.02.92 Bulletin 92/09**

(84) Etats contractants désignés :
**DE GB IT NL**

(71) Demandeur : **INSTITUT FRANCAIS DU PETROLE**
**4, avenue de Bois-Préau**
**F-92502 Rueil-Malmaison (FR)**

(72) Inventeur : **Raatz, Francis**
**25, rue de la Carrière**
**F-57500 Saint-Avold (FR)**
Inventeur : **Guth, Jean-Louis**
**59, rue Bellevue, Brunstatt**
**F-68200 Mulhouse (FR)**
Inventeur : **Bournonville, Jean-Paul**
**43, rue des Groues, Vauréal**
**F-95000 Cergy Pontoise (FR)**
Inventeur : **Juguin, Bernard**
**46, avenue du Stade**
**F-92500 Rueil Malmaison (FR)**
Inventeur : **Joly, Jean-François**
**20, rue Béranger**
**F-75003 Paris (FR)**

(54) **Utilisation d'un catalyseur de type galloalumino-silicate en aromatisation des hydrocarbures contenant 5 à 7 atomes de carbone par molécule.**

(57) L'invention concerne l'utilisation, pour l'aromatisation d'hydrocarbures comportant 5 à 7 atomes de carbone par molécule, d'un catalyseur de type galloaluminosilicate renfermant en poids :

a) 0,01 à 99,49 % d'une matrice choisie dans le groupe formé par l'alumine, la silice, la magnésie, une argile et toute combinaison d'au moins deux des composés précités et

b) 0,51 à 99,99 % d'une zéolithe synthétisée en milieu fluorure de formule chimique approchée suivante :

$$(M_{(x+y)/n})^{(x+y)+} \ (Si_{(96-(x+y))} \ Al_x Ga_y O_{192})^{(x+y)-}$$

M représente un proton et/ou un cation métallique
x est compris entre 0,1 et 9
y est compris entre 0,1 et 7
la zéolithe ayant une teneur en fluor comprise entre 0,02 et 1,5 % en poids, le fluor étant incorporé lors de la synthèse, ladite zéolithe étant aussi caractérisée par un diagramme de diffraction X particulier.

EP 0 472 462 A1

La présente invention concerne l'utilisation dans les réactions d'aromatisation des hydrocarbures contenant entre 5 et 7 atomes de carbone par molécule, en présence ou non d'oléfines, d'un catalyseur de type galloaluminosilicate comprenant une zéolithe de structure MFI, synthétisée en milieu fluorure, contenant du silicium, de l'aluminium, et du gallium.

La synthèse dans des milieux fluorures de zéolithes de structure MFI a déjà été décrite dans le brevet français n° 2567868 pour le système Si-Al et dans le brevet européen EP-B-342075 pour le système Si-Ga. Une nouvelle synthèse en milieu fluorure de zéolithe de structure MFI dans le système Si-Ga-Al consiste :

a) dans une première étape à préparer un mélange réactionnel en solution à un pH inférieur ou égal à environ 10 comprenant, notamment de l'eau, au moins une source de silicium, au moins une source d'aluminium, au moins une source de gallium trivalent, au moins une source d'au moins un ion fluorure $F^-$ et une source d'au moins un agent structurant fournissant des cations organiques contenant de l'azote. L'agent structurant est choisi parmi les di-, trialkylamines et les cations ammonium dérivant par protonation des dites amines, et/ou les cations tétraalkylammonium, les groupes alkyl étant de préférence les groupes n-propyl. Ledit mélange a une composition en termes de rapports molaires comprise dans les intervalles suivants :

$Si^{IV}/Al^{III}$ : 2-1000

$Si^{IV}/Ga^{III}$ : 2-1000

$F^-/Si^{IV}$ : 0,05-3

structurant organique/$Si^{IV}$ : 0,04-1

$H_2O/Si^{IV}$ : 4-400

et un pH entre 2 et 11 (de préférence entre 4 et 10),

b) dans une deuxième étape à chauffer ledit mélange à une température au plus égale à environ 270°C, de manière avantageuse entre 80 et 220°C et de préférence entre 140 et 210°C, pendant une durée suffisante pour obtenir des cristaux de galloaluminosilicate,

c) dans une troisième étape à calciner les dits cristaux à une température supérieure à 400°C et de préférence comprise entre 500 et 600°C. L'étape de calcination a pour but l'élimination des cations organiques ou ammonium contenus dans le solide brut de synthèse.

Le pH inférieur à 10 du milieu réactionnel peut être obtenu soit directement à partir de l'un ou plusieurs des produits composant le milieu réactionnel, soit par l'ajout audit milieu d'un acide, d'une base, d'un sel acide, d'un sel basique ou d'un mélange tampon complémentaire.

Les anions fluorures $F^-$ peuvent être introduits dans le milieu réactionnel sous forme de fluorures, comme le fluorure de sodium NaF, le fluorure d'ammonium $NH_4F$, le fluorure acide d'ammonium $NH_4HF_2$, le fluorure de tétrapropylammonium $(C_3H_7)_4NF$, le fluorure de tétrapropylphosphonium $(C_3H_7)_4PF$, ou de composés hydrolysables pouvant libérer des anions fluorures dans l'eau, comme le fluorure de silicium $SiF_4$ ou le fluorosilicate de sodium $Na_2SiF_6$.

Le fluorure d'ammonium ou le fluorure d'ammonium acide sont des sels préférés, car ils permettent l'obtention d'une zéolithe de structure MFI facile à transformer en sa forme protonée sans qu'il y ait lieu de procéder à des réactions d'échange d'ions.

De nombreuses sources de silice peuvent être utilisées dans la formation du milieu réactionnel. On peut citer, par exemple :

– les silices sous forme d'hydrogels, d'aérogels, de suspensions colloïdales,

– les silices résultant de la précipitation de solutions de silicates solubles, ou de l'hydrolyse d'esters siliciques comme l'ester tétraéthylique de l'acide monoorthosilicique $Si(OC_2H_5)_4$, ou de complexes comme le fluorosilicate de sodium $Na_2SiF_6$ ou d'ammonium $(NH_4)_2SiF_6$,

– les silices préparées par des traitements d'extraction et d'activation de composés cristallisés naturels ou synthétiques, comme les silicates d'aluminium, les aluminosilicates, les argiles, etc...

Les silices utilisées peuvent être divisées ou agglomérées.

Parmi les sources utilisables on peut citer les sels d'aluminium (sulfate, nitrate, chlorure, fluorure, acétate par exemple), les hydroxydes et oxydes d'aluminium, les aluminates, les esters comme l'ester tripropylique de l'acide monoorthoaluminique $Al(OC_3H_7)_3$.

Au lieu de partir de sources séparées d'alumine et de silice, on peut aussi utiliser des sources où les deux oxydes sont combinés, comme, par exemple, les gels de silice-alumine amorphes, les aluminosilicates cristallisés, parmi lesquels on peut citer les argiles et les zéolithes.

Les sources de silice et d'alumine peuvent être engagées sous forme soluble ou solide, mais également sous forme d'agglomérats comme des extrudés ou des pastilles. Ce dernier conditionnnement convient bien aux sources à base de zéolithes brutes ou modifiées déjà agglomérées, qui peuvent ainsi être transformées, selon le nouveau procédé, en zéolithes préformées.

Les sources de l'élément $Ga^{III}$ utilisées sont, par exemple :

– les sels de gallium (sulfate, nitrate, chlorure, fluorure, acétate par exemple),

– les hydroxydes, les hydroxyoxydes et oxydes de gallium, les gallates et différents esters.

Il est également possible d'utiliser les sources contenant les éléments silicium et gallium associés tels par exemple des verres ou des co-gels.

Les sources des éléments silicium et gallium peuvent être engagées sous forme de fluides ou de solides pulvérulents, mais également sous forme d'agglomérats, tels que par exemple pastilles ou extrudés, pouvant être transformés en zéolithes sans modification de la forme.

Il est possible d'utiliser des sources où les éléments gallium et aluminium sont associés comme par exemple des gels obtenus par co-précipitation d'hydroxyde ou d'oxyhydroxydes de gallium et d'aluminium.

Les sources pour lesquelles les éléments gallium et aluminium sont associés peuvent être engagés sous forme de liquides ou de solides pulvérulents, mais également sous forme d'agglomérats, tels par exemple, pastilles ou extrudés, pouvant être transformés en zéolithes sans modification de forme.

Il est également possible d'utiliser des sources où les éléments gallium, aluminium et silicium sont associés comme par exemple des gels amorphes, des verres ou des galloaluminosilicates cristallisés parmi lesquels on peut citer les argiles et les zéolithes.

Les sources des éléments gallium, aluminium et silicium peuvent être engagés sous forme de liquides ou de solides pulvérulents, mais également sous forme d'agglomérats, tels que, par exemple, pastilles ou extrudés, pouvant être transformés en zéolithes sans modification de forme.

En utilisant des sources de silicium, aluminium, gallium séparées ou non on peut régler la répartition de l'aluminium et du gallium au sein des cristaux. Cette répartition peut aussi être réglée par le rapport Ga/Al et F/Ga+Al+Si, on peut obtenir des solides contenant un coeur riche en gallium et une zone externe riche en aluminium ou au contraire des solides pour lesquels la répartition aluminium gallium est parfaitement homogène.

En effet l'introduction d'éléments trivalents $T^{III}$ ($Al^{III}$ et $Ga^{III}$) dépend du rapport $Si^{IV}/T^{III}$ dans le mélange réactionnel. Mais elle dépend aussi de leur disponibilité et de leur aptitude à être incorporé dans la zéolithe.

La disponibilité est fonction de la nature des sources contenant les éléments Si, Al et Ga. L'aptitude à être incorporée dans la zéolithe est fonction de la nature des espèces chimiques sous lesquelles elles sont présentes dans la solution du mélange réactionnel. En particulier un nombre trop élevé de ligands $F^-$ par rapport aux ligands OH polycondensables (fonction du rapport F/Si+Al+Ga du mélange réactionneel et de la stabilité des complexes fluorés des éléments $T^{III}$) rend l'incorporation des éléments $Al^{III}$ et $Ga^{III}$ plus difficile (la stabilité de ces complexes fluorés décroît dans le sens Al>Ga).

Ainsi si on désire obtenir une zéolithe contenant le gallium essentiellement dans le coeur des cristaux il faut synthétiser des cristaux, dont le rapport $Si^{IV}/Ga^{III}$ est supérieur à 25, à partir d'un mélange réactionnel ayant un rapport $F^-/(Si^{IV}+Ga^{III})$ pas trop élevé et contenant tout le gallium dans la phase aqueuse, l'essentiel de la source de silicium étant une source solide telle que la silice $SiO_2$.

L'aluminium sera incorporé dans la zone externe des cristaux, lorsque dans le mélange réactionnel précédent, on remplace la source de silice solide par une source de silice-alumine solide ou si on rajoute l'aluminium sous la forme d'un sel d'un oxyde ou hydroxyde.

Dans les deux synthèses précédentes on peut étendre la zone interne occupée par le gallium en utilisant un mélange réactionnel dans lequel le rapport $F^-/(Si^{IV}+Ga^{III}+Al^{III})$ a une valeur plus élevée.

Lorsqu'on veut obtenir des cristaux dans lesquels la répartition en $Al^{III}$ et $Ga^{III}$ est plus homogène il faut utiliser de préférence une source où les éléments Si, Al et Ga sont associés et un rapport Al/Ga de préférence supérieur à 1 pour compenser l'effet de la plus forte rétention de $Al^{III}$ en solution sous forme de complexes fluorés difficilement incorporables.

Les sources d'agent structurant pouvant fournir des cations organiques sont de préférence des cations tétrahydrocarbylammonium, tétrahydrocarbylphosphonium, hydrocarbyl étant avantageusement alkyl et de manière préférée propyle.

Les cations tétrapropylammonium ($TPA^+$) ou tétrapropylphosphonium ($TPP^+$) qui sont les agents structurants préférés sont ajoutés de préférence sous forme de leurs sels, par exemple les bromures, les fluorures, mais ils peuvent également être engendrés in situ à partir de tripropylamine ou de tripropylphosphine et d'un halogénure de propyle.

Les acides ou sels acides, les bases ou sels basiques ajoutés éventuellement en complément pour amener le pH du milieu réactionnel à la valeur désirée peuvent être choisis parmi les acides courants, comme l'acide fluorhydrique HF, l'acide chlorhydrique HCl, l'acide nitrique $HNO_3$, l'acide sulfurique $H_2SO_4$, l'acide acétique $CH_3COOH$, ou les sels acides comme le fluorure acide d'ammonium $NH_4HF_2$, le florure acide de potassium $KHF_2$, le sulfate acide de sodium $NaHSO_4$, le sulfate acide de potassium $KHSO_4$, le phosphate acide de sodium $NaH_2PO_4$ et les bases courantes comme l'ammoniaque $NH_4OH$, la soude NaOH, la potasse KOH ou les sels basiques courants comme les carbonates acides $NaHCO_3$ ou neutre $Na_2CO_3$ de sodium, l'acétate de sodium $CH_3COONa$, les sulfures neutres $Na_2S$ ou acide NaHS de sodium ou les mélanges tampons comme acide acétique CH₃COOH-acétate de sodium CH₃COONa, ammoniaque NH₄OH-chlorure d'ammonium NH₄Cl.

La morphologie, la taille et la cinétique de formation de cristaux de zéolithes obtenues selon le procédé de l'invention peuvent être modifiées par l'introduction dans le milieu réactionnel de sels complémentaires comme le chlorure de sodium NaCl, le chlorure de potassium KCl, le chlorure d'ammonium NH₄Cl, le sulfate de sodium Na₂SO₄ et/ou de cristaux (broyés ou non) de composés solides apparentés aux zéolithes préparées par le procédé de l'invention.

L'ajout de cristaux (germes) au mélange réactionnel et l'agitation facilitent généralement la cristallisation et ont également une influence sur la taille des cristaux de zéolithes formés.

Le chauffage du mélange réactionnel est fait de préférence dans un autoclave revêtu intérieurement de polytétrafluoréthylène (PTFE). Suivant la composition, l'ajout de germes, la température et l'agitation ou non, la durée de chauffage se situe généralement entre 6 et 650 heures. Lorsque la cristallisation est achevée, le solide obtenu est filtré et lavé à l'eau désionisée.

Les zéolithes de type galloaluminosilicate obtenues par le procédé selon l'invention sont identifiées de manière commode à partir de leur diagramme de diffraction des rayons X. Il peut être obtenu à l'aide d'un diffractomètre en utilisant la méthode des poudres avec le rayonnement K alpha du cuivre. Les équidistances réticulaires $d_{hkl}$ caractéristiques de l'échantillon sont calculées par la relation de Bragg, à partir de la position des pics de diffraction, représentée par l'angle 2 thêta. L'estimation de l'erreur de mesure $d_{hkl}$ se calcule en fonction de l'erreur absolue 2 thêta affectée à la mesure de 2 thêta par la relation de Bragg. L'erreur absolue 2 thêta, couramment admise, est égale à +- 0,2°. L'intensité relative I/Io, I étant l'intensité d'une raie donnée et Io l'intensité de la raie la plus forte, affectée à chaque valeur de $d_{hkl}$ est estimée à partir de la hauteur du pic de diffraction correspondant. Les tableaux 1 et 2 ci-après représentent les diagrammes de diffraction des rayons X caractéristiques de deux zéolithes de type galloaluminosilicate A et B obtenues selon le procédé de la présente invention, calcinés à 550°C, sous air, le rapport molaire $Si^{IV}/T^{III}$ ($T^{III}$ = Al et/ou Ga) de A est au plus égal à 50, le rapport molaire $Si^{IV}/T^{III}$ de B est au moins égal à 50. Les colonnes de $d_{hkl}$ des tableaux 1 et 2 représentent les valeurs extrêmes que peuvent prendre les différentes équidistances $d_{hkl}$. Les valeurs dépendent du rapport $Si^{IV}/T^{III}$ et de la nature des cations de compensation, chacune des valeurs indiquées dans les tableaux doit encore être affectée de l'erreur de mesure $d_{hkl}$. Pour caractériser les intensités relatives I/Io, une échelle de symbole est souvent utilisée : FF : très fort, F = fort, mF = moyen à fort, m = moyen, mf = moyen à faible, f = faible, ff = très faible. Ces intensités relatives dépendent également en partie de la composition des zéolithes de type gallosilicate obtenues.

Les solides obtenus par la procédure de synthèse décrite précédemment sont des zéolithes de structure MFI qui ont après calcination comme formule chimique approchée de la maille unité :

$$(M_{(x + y)/n})^{(x + y) +} \ (Si_{(96-(x + y))} Al_x Ga_y O_{192})^{(x + y)-}$$

où x peut varier de 0,1 à 9, y peut varier entre 0,1 et 7 et où M représente le ou les cations de compensation de valence n. Le point important est que ces solides contiennent, après l'étape de synthèse et également après l'étape d'élimination des composés organiques, de l'élément fluor. La teneur en fluor dans la zéolithe déterminée par analyse élémentaire est comprise pour les solides calcinés, c'est-à-dire ceux résultant de l'étape (c) décrite précédemment, entre 0,02 et 1,5 % en poids, avantageusement entre 0,05 et 1,0 % et de préférence entre 0,2 et 0,8 %.

Les zéolithes du type galloaluminosilicate utilisées dans la présente invention possèdent des propriétés acides tout à fait différentes de celles des zéolithes de structure MFI classiques obtenues en milieu alcalin (PCT WO84/03879, EP 0299392, J. Kanai and N. Kawata, Appl. Catal., 55, (1989), p. 115-122). C'est la présence de fluor et plus spécialement la procédure par laquelle le fluor est incorporé dans les solides qui sont responsables de ces propriétés acides particulières ainsi qu'il a été expliqué dans le brevet européen EP-B-342075 et le brevet européen EP-B-351311.

L'acidité particulière des solides résultant de l'introduction de fluor lors de la synthèse, est la particularité mise à profit pour préparer des catalyseurs d'aromatisation susceptibles par exemple d'aromatiser un hydrocarbure contenant 5 à 7 atomes de carbone en présence ou non d'oléfines et dont les propriétés acides sont d'un type nouveau.

La présente invention concerne donc l'utilisation d'un catalyseur du type galloaluminosilicate caractérisé par la composition suivante exprimée en poids :

a) 0,01 à 99,49 % d'une matrice choisie dans le groupe formé par l'alumine, la silice, la magnésie, une argile et toute combinaison d'au moins deux des composés précités et

b) 0,51 à 0,99 % d'une zéolithe synthétisée en milieu fluorure de formule chimique approchée suivante :

$$(M_{(x + y)/n})^{(x + y) +} \ (Si_{(96-(x + y))} Al_x Ga_y O_{192})^{(x + y)-}$$

M représente un proton et/ou un cation métallique

x est compris entre 0,1 et 9

y est compris entre 0,1 et 7

la zéolithe ayant une teneur en fluor comprise entre 0,02 et 1,5 % en poids, le fluor étant incorporé lors de la synthèse, ladite zéolithe étant aussi caractérisée par un diagramme de diffraction X présenté dans les tableaux 1 ou 2.

De façon plus précise après synthèse en milieu fluorure, le solide peut être soumis si besoin est, à un traitement au moins partiel de défluoration permettant d'ajuster ses propriétés acides.

La zéolithe synthétisée renfermant du fluor et de type galloaluminosilicate de structure MFI, est caractérisée par :

- une teneur en fluor comprise entre 0,02 % et 1,5 % en poids,
- un rapport molaire $Si^{IV}/Ga^{III}$ supérieur ou égal à 13,
- un rapport molaire $Si^{IV}/Al^{III}$ supérieur ou égal à 10.

Le traitement de défluoration est plus ou moins sévère suivant le niveau de défluoration désiré. Il consiste en un ou plusieurs traitements successifs du solide, à reflux dans une solution d'ammoniaque de normalité comprise entre environ 0,05 et 5N et de préférence entre 0,1 et 3N, pendant une durée comprise entre environ 0,5 et 5 heures et de préférence entre 1 et 4 heures avec un rapport v/p défini comme le volume de solution par rapport au poids de solide sec compris entre environ 5 et 50 $cm^3g^{-1}$ et de préférence entre 10 et 30 $cm^3g^{-1}$. Le solide, après chaque lavage est ensuite abondamment lavé à l'eau distillée et séché à l'étuve. Après ces traitements et suivant leur sévérité, la teneur en fluor du solide est comprise entre 0,9 et 0,01 % en poids. Si on élimine, par des traitements répétés, sensiblement la totalité du fluor, on aboutit encore à des solides qui se distinguent en particulier par leur spectre IR dans la région 3800-3500 $cm^{-1}$ des zéolithes de structure MFI classiques de même rapport Si/Ga de charpente : les solides contenus dans le catalyseur selon l'invention possèdent une proportion plus importante de groupes Si-OH.

Le solide partiellement ou totalement défluoré peut être mélangé à une matrice généralement amorphe, par exemple à une poudre humide de gel d'alumine. Le mélange est ensuite mis en forme, par exemple par extrusion au travers d'une filière. La teneur en zéolithe du support ainsi obtenu est généralement comprise entre environ 0,5 et 99,99 % et avantageusement comprise entre environ 40 et 90 % poids. Elle est plus particulièrement comprise entre environ 60 et 85 % en poids par rapport à l'ensemble zéolithe et matrice.

La teneur en matrice du catalyseur est avantageusement comprise entre environ 10 et 60 % et de préférence entre environ 15 et 40 % en poids. La mise en forme peut être réalisée avec d'autres matrices que l'alumine, telles que, par exemple la magnésie, la silice-alumine, les argiles naturelles (kaolin, bentonite), et par d'autres techniques que l'extrusion telles que le pastillage, la dragéification ou la coagulation en goutte.

La zéolithe, partiellement défluorée ou non, peut essentiellement avant ou après la mise en forme avec une matrice amorphe, être taitée en présence de vapeur d'eau à haute température. Ce traitement, déjà préconisé dans le cas des gallosilicates synthétisés en milieu alcalin (WO 84/03879), augmente avantageusement les propriétés catalytiques du solide pour la réaction d'aromatisation d'une coupe $C_2$-$C_4$. Ce traitement consiste en une calcination conduite sous un gaz (air ou gaz contenant de l'air ou un gaz inerte), ce gaz contenant de préférence entre 5 % et 100 % vapeur d'eau, à une température située entre 400 et 900°C, de préférence entre 450 et 600°C.

Ce traitement n'est en fait absolument pas indispensable. En effet le catalyseur préparé à partir de la zéolithe selon l'invention est destiné à être utilisé dans un procédé faisant appel à des régénérations fréquentes. Ces régénérations sont réalisées classiquement à des températures comprises entre 450 et 650°C. Ainsi l'action de ces hautes températures va conduire inévitablement à une évolution du solide, évolution qui a pour effet d'améliorer ses performances catalytiques. Sans nous lier à une théorie particulière, il est probable que cette amélioration est due à une extraction partielle ou totale du gallium de la charpente, celui-ci occupant alors des positions hors charpente. Ainsi comme en craquage catalytique, des prétraitements à haute température du catalyseur en présence de vapeur d'eau peuvent tout à fait être évités, puisque le catalyseur évoluera naturellement au cours des régénérations successives vers une forme que l'on peut qualifier de forme équilibrée. C'est cette forme équilibrée qui conduit aux performances catalytiques optimales.

L'effet cumulé de ces traitements de régénération et de la présence de fluor apporte au solide un nouveau type d'acidité qui conduit à un catalyseur d'aromatisation des hydrocarures en $C_5$-$C_7$ dont les performances sont améliorées par rapport aux catalyseurs de l'art antérieur ne contenant pas de fluor.

Le catalyseur obtenu par les procédures de préparation précédentes est mis en oeuvre pour la réaction d'aromatisation des hydrocarbures contenant 5 à 7 atomes de carbone par molécule en présence ou non d'oléfines. Cette réaction revêt un intérêt particulier car elle permet de valoriser des résidus d'opérations de raffinage en des produits à plus haute valeur ajoutée (benzène, toluène, xylène) tout en contribuant à la production de quantités importantes d'hydrogène indispensable pour les procédés d'hydrotraitement par exemple.

La charge est mise en contact avec le catalyseur préparé suivant l'invention à une température comprise entre 400°C et 700°C et plus particulièrement entre 480 et 580°C. En réalité la température de réaction est à adapter à la charge à traiter.

Les exemples qui suivent précisent l'invention sans toutefois en limiter la portée. Ils sont donnés pour quelques charges typiques, toutefois ils sont facilement transposables à des charges complexes.

TABLEAU 1

DIAGRAMME DE DIFFRACTION RX DES GALLOALUMINOSILICATES B CALCINES
A STRUCTURE MONOCLINIQUE SELON L'INVENTION
(les mesures de $d_{hkl}$ ont été arrêtées à des valeurs de l'ordre de
2,97 Å, il est néanmoins possible d'effectuer des mesures de $d_{hkl}$
pour des valeurs de 2 thêta plus grandes)

| $d_{hkl}$ Å $(10^{-10}$ m) | I/Io | $d_{hkl}$ Å $(10^{-10}$ m) | I/Io |
|---|---|---|---|
| 11,00-11,15 | FF | 4,22-4,27 | f |
| 9,99- 9,86 | F | 4,04-4,09 | ff |
| 9,67- 9,79 | m | 3,97-4,02 | ff |
| 8,89- 9,00 | fff | 3,90-3,95 | fff |
| 7,95- 8,06 | fff | 3,82-3,87 | F |
| 7,35- 7,44 | fff | 3,79-3,84 | mF |
| 6,99- 7,09 | fff | 3,77-3,82 | mF |
| 6,62- 6,71 | f | 3,72-3,77 | m |
| 6,29- 6,37 | f | 3,70-3,75 | m |
| 5,92- 6,00 | mf | 3,69-3,73 | m |
| 5,85- 5,93 | f | 3,63-3,6 | ff |
| 5,67- 5,74 | f | 3,60-3,65 | ff |
| 5,63- 5,70 | f | 3,42-3,46 | fff |
| 5,52- 5,59 | f | 3,32-3,37 | fff |
| 5,32- 5,39 | ff | 3,28-3,32 | ff |
| 5,09- 5,15 | fff | 3,23-3,27 | fff |
| 4,98- 5,04 | f | 3,03-3,07 | ff |
| 4,93- 4,99 | f | 3,01-3,05 | ff |
| 4,57- 4,63 | ff | 2,96-3,00 | f |
| 4,32- 4,38 | ff | 2,93-2,97 | ff |

## TABLEAU 2

### DIAGRAMME DE DIFFRACTION RX DES GALLOALUMINOSILICATES A CALCINEES A STRUCTURE ORTHORHOMBIQUE SELON L'INVENTION

| $d_{hkl}$ $(10^{-10}$ m) Å | I/Io | $d_{hkl}$ $(10^{-10}$ m) Å | I/Io |
|---|---|---|---|
| 11,03-11,18 | FF | 4,32-4,38 | ff |
| 9,90-10,03 | F | 4,22-4,28 | f |
| 9,65- 9,78 | m | 4,05-4,11 | fff |
| 8,88- 9,00 | fff | 3,97-4,02 | ff |
| 7,93- 8,03 | fff | 3,82-3,87 | F |
| 7,36- 7,45 | fff | 3,78-3,83 | mF |
| 6,99- 7,09 | fff | 3,72-3,77 | m |
| 6,63- 6,72 | ff | 3,69-3,74 | m |
| 6,30- 6,38 | f | 3,62-3,67 | m |
| 5,93- 6,01 | mf | 5,56-3,61 | fff |
| 5,89- 5,97 | f | 3,41-3,45 | ff |
| 5,65- 5,72 | f | 3,32-3,37 | ff |
| 5,52- 5,59 | f | 3,29-3,33 | ff |
| 5,32- 5,39 | ff | 3,23-3,27 | fff |
| 5,07- 5,14 | fff | 3,03-3,07 | ff |
| 4,97- 5,04 | ff | 2,96-3,00 | f |
| 4,93- 4,99 | ff | 2,94-2,97 | ff |
| 4,57- 4,63 | ff | | |

Les mesures de $d_{hkl}$ ont été arrêtées à des valeurs de l'ordre de 2,97 Å, il est néanmoins possible d'effectuer des mesures de $d_{hkl}$ pour des valeurs de 2 thêta plus grandes.

## EXEMPLE 1

L'exemple 1 illustre, par 4 synthèses de galloaluminosilicates du type MFI, comment la méthode de synthèse en milieu fluorure permet d'obtenir des zéolithes de compositions variables mais où l'essentiel du gallium est présent au coeur des cristaux et l'essentiel de l'aluminium dans la zone périphérique (répartition Ga$^{III}$, Al$^{III}$ hétérogène).

Chaque mélange réactionnel est préparé en mélangeant de la silice Aerosil 130, du chlorure de gallium, du chlorure d'aluminium, du bromure de tétrapropylammonium (TBA Br) du fluorure d'aluminium et de l'eau. La composition des différents mélanges réactionnels est indiquée dans le tableau 3 sous forme de rapports molaires. La température de chauffage du mélange réactionnel dans un autoclave avec un revêtement interne en polytétrafluoroéthane, est de 200°C. La durée du chauffage ainsi que la présence éventuelle de germes (% pondéral de cristaux broyés par rapport au poids de silice engagée) sont précisés dans le tableau 3. Après chauffage à l'autoclave la phase solide obtenue est filtrée, lavée à l'eau et séchée à 80°C. Dans le tableau 4 figurent les résultats concernant la cristallinité (% MFI), la morphologie des cristaux ainsi que la composition chimique globale en gallium et aluminium (exprimée en rapport $Si^{IV}/T^{III}$) ainsi que les mêmes valeurs de $Si^{IV}/T^{III}$ mesurées au coeur et en périphérie des cristaux (mesures effectuées à l'aide d'une analyse par microsonde électronique sur des coupes de cristaux).

TABLEAU 3

CONDITIONS OPERATOIRES POUR LES SYNTHESES 1a A 1d

| Essai | Si/Ga | Si/Al | F/Si | TPA$^+$/Si | H$_2$O/ Si | Germes (2 %) | Statique (1) Agité(a) | pH initial et final | Durée (jours) |
|-------|-------|-------|------|-----------|------|--------|-----------|-----------|--------|
| 1a | 80 | 82 | 0,5 | 0,25 | 50 | oui | 1 | 6-6,5 | 5 |
| 1b | 51 | 49 | 0,5 | 0,25 | 50 | non | 1 | 6,5-6,5 | 21 |
| 1c | 20 | 20 | 0,5 | 0,25 | 50 | oui | a | 4-5 | 4 |
| 1d | 100 | 20 | 0,5 | 0,25 | 50 | non | 1 | 6-6 | 54 |

TABLEAU 4

RESULTATS DES ANALYSES DES ECHANTILLONS 1a A 1d

| Essai | % MFI | Tailles moyennes | Si/Ga global | Si/Al global | Si/Ga coeur | Si/Al coeur | Si/Ga périphérie | Si/Al périphérie |
|-------|-------|----------|---------|---------|------|------|-----------|-----------|
| 1a | 100 | 4x5x3µm | 76 | 114 | 30 | 150 | 130 | 50 |
| 1b | 90 | 90x30µm | 51 | 95 | 22 | 250 | 85 | 70 |
| 1c | 100 | 15x10x5µm | 26 | 200 | 20 | 300 | 70 | 60 |
| 1d | 100 | 60x25x 20µm | 76 | 95 | 25 | 150 | 200 | 42 |

Exemple 2

Les 4 synthèses de l'exemple 2 montrent que l'on peut rendre la répartition de Al$^{III}$ et Ga$^{III}$ plus homogène dans les cristaux de galloaluminosilicates du type MFI en jouant sur la nature des sources apportant les éléments Si, Al et Ga et sur le rapport Al/Ga engagé.

– La source A est obtenue avec un aluminosilicate amorphe (Tixolex 28 de la Société Poulenc, Si/Al = 7,3) dont les cations $Na^+$ ont été préalablement échangés contre des cations $NH_4^+$ et auquel on a jouté une solution de chlorure de gallium (Si/Ga= 50).

– La source B est un xérogel formé de la façon suivante. On effectue l'hydrolyse acide (pH = 1) sous reflux et sous agitation de tétraorthosilicate d'éthyl $Si(OC_2H_5)_4$ avec une solution aqueuse contenant du chlorure de gallium et du chlorure d'aluminium. Le rapport $H_2O/Si(OC_2H_5)$ est d'environ 30, le rapport Al/Ga est de 1 et le rapport Si/(Al+Ga) est de 25. L'hydrolyse terminée on ajoute lentement sous agitation de l'ammoniaque concentré jusqu'à ce que le pH de la solution soit voisin de 7. Le gel ainsi obtenu est séché à 70°C.

– La source C est un xérogel formé comme la source B mais en engageant un rapport Al/Ga = 5 et un rapport Si/(Al+Ga) de 5.

– La source D est un xérogel formé comme la source B mais en engageant uniquement une solution de chlorure de gallium (rapport Si/Ga = 30). Après formation et séchage du xérogel on lui ajoute une solution aqueuse de chlorure d'aluminium telle que le rapport global Si/(Al+Ga) = 5.

Pour préparer les 4 mélanges réactionnels on ajoute à chacune des sources d'éléments Si, Al, Ga préparées ci-dessus du fluorure d'aluminium, du bromure de tétrapropylammonium et éventuellement un complément d'eau de manière à obtenir les rapports molaires figurant dans le tableau 5. Les 4 mélanges réactionnels sont ensuite chauffés dans des autoclaves agités, comportant un revêtement-interne en polytétrafluoroéthane à 200°C pendant 15 jours. Après refroidissement, les solides formés sont filtrés, lavés à l'eau et séchés à 80°C.

## TABLEAU 5

### COMPARAISON EN RAPPORT MOLAIRE DES MELANGES REACTIONNELS DE L'EXEMPLE 2

| Essai | Source | Si/Ga | Si/Al | Al/Ga | Si/Al+Ga | F/Si | $TPA^+$/Si | $H_2O$/Si |
|-------|--------|-------|-------|-------|----------|------|--------|---------|
| 2a | A | 50 | 7,3 | 6,6 | 28,5 | 0,5 | 0,25 | 50 |
| 2b | B | 50 | 50 | 1 | 25 | 0,5 | 0,25 | 50 |
| 2c | C | 30 | 6 | 5 | 5 | 0,5 | 0,25 | 50 |
| 2d | D | 30 | 6 | 5 | 5 | 0,5 | 0,25 | 50 |

L'analyse radiocristallographique montre que les solides ainsi obtenus sont du type MFI avec une cristallinité supérieure à 90 %. La dimension des cristaux varie entre 5 et 30 μm.

Le tableau 6 fournit les valeurs des rapports Si/Ga et Si/Al déterminés par analyse chimique globale des cristaux ainsi que les valeurs de ces rapports déterminés au coeur et en périphérie des cristaux à l'aide d'une microsonde électronique sur des coupes de cristaux.

## TABLEAU 6

### RAPPORTS Si/Ga et Si/Al GLOBAUX, AU COEUR ET EN PERIPHERIE DES CRISTAUX

| Essai | Si/Ga global | Si/Al global | Si/Ga au coeur | Si/Al au coeur | Si/Ga en périphérie | Si/Al en périphérie |
|-------|--------------|--------------|----------------|----------------|---------------------|---------------------|
| 2a | 49 | 39 | 35 | 47 | 60 | 36 |
| 2b | 54 | 62 | 40 | 70 | 60 | 45 |
| 2c | 35 | 30 | 30 | 40 | 45 | 25 |
| 2d | 40 | 25 | 35 | 50 | 45 | 20 |

### EXEMPLE 3 : CATALYSEURS CONFORMES A L'INVENTION

A partir des solides 1a, 1b synthétisés conformément à l'exemple 1, et des solides 2a, 2b synthétisés conformément à l'exemple 2, on prépare des catalyseurs d'aromatisation de la manière suivante :

i) après synthèse les solides 1a, 1b, 2a, 2b sont calcinés sous air sec à 570°C pendant 3 heures,

ii) les solides calcinés sont mis en forme par extrusion avec un gel d'alumine à raison de 25 % pds de liant pour 75 % de zéolithe,

iii) les solides extrudés sont calcinés à 560°C sous air sec pendant 2 heures.

Les extrudés calcinés ainsi obtenus sont testés en aromatisation du propane dans les conditions suivantes :

i) traitement initial sous azote à 500°C pendant 2 heures,

ii) passage sous charge de pentane (iC$_5$ 60 % ; nC$_5$ 40 %) à 520°C avec une PPH de 6 h-1 pendant 6 heures sous une pression de 1 bar,

iii) purge sous azote à 550°C pendant 2 heures,

iv) régénération à 510°C sous un mélange d'oxygène dilué à 5 % dans l'azote pendant 4 heures, puis à 550° sous un mélange d'oxygène dilué à 15 % dans l'azote pendant 4 heures,

v) purge sous azote à 550°C pendant 2 heures.

A l'issue de la purge sous azote qui suit la régénération, les extrudés sont de nouveau soumis au passage de la charge de pentane pur à 490°C avec une PPH de 6 h-1 sous une pression de 1 bar. Afin d'aboutir à un état pour lequel les performances des catalyseurs n'évoluent plus entre chaque phase de réaction, on leur fait subir 10 cycles réaction-régénération.

Les performances des catalyseurs obtenus après au moins 10 cycles réaction - régénération sont données dans le tableau 7. Avec ces catalyseurs on obtient des sélectivités en aromatiques par passe qui sont supérieures à 52 %, et qui peuvent même largement dépasser 59 % pour les catalyseurs 2a et 2b. Ces résultats indiquent qu'il est préférable de préparer des solides pour lesquels les répartitions en aluminium et gallium au sein des cristaux est aussi homogène que possible après la synthèse. Il faut noter que les teneurs en fluor des solides mesurés après le traitement sont importantes (tableau 7).

### EXEMPLE 4

La préparation d'un catalyseur à partir de la zéolithe 2a défluorée de la présente invention illustre l'importance du fluor sur les propriétés catalytiques en aromatisation du propane.

On utilise comme zéolithe de départ la zéolithe 2a de l'exemple 2. Après synthèse, cette zéolithe est calcinée sous air sec à 570°C pendant 4 heures puis est soumise à un traitement de défluoration qui consiste en trois cycles :

– traitement dans $NH_4OH$ 0.35 N à 100°C pendant 2 heures,
– filtration et lavage à l'eau distillée,
– séchage à l'étuve à 120°C une nuit.

Après ce traitement, on obtient un solide dont la cristallinité, les rapports Si/Al et Si/Ga n'ont pas été modifiés mais dont la teneur en fluor est inférieure à la limite de détection de notre méthode de dosage, c'est-à-dire 0.02 % pds.

Le solide défluoré ainsi obtenu, référencé 2adf est testé en aromatisation dans les conditions de l'exemple 3, c'est-à-dire après un prétraitement sous azote suivi d'un cycle de réaction -régénération. Les performances de ce catalyseur reportées dans le tableau 7 sont inférieures, particulièrement du point de vue activité, à celles de son homologue non défluoré 2a.

EXEMPLE 5 : Catalyseur de comparaison CI

On prépare un galloaluminosilicate de structure MFI en milieu conventionnel basique suivant les procédures connues dans l'art antérieur (brevet Européen : 0 299 392). La zéolithe MFI ainsi obtenue est caractérisée par un rapport Si/Al égal à 55 et un rapport Si/Ga égal à 100. Après passage à la forme hydrogène par une calcination sous air à 550°C suivie de trois échanges ioniques dans $NH_4NO_3$ 0.5 N à 90°C, et enfin calcination finale à 550°C pendant 3 heures sous air, on prépare un catalyseur d'aromatisation référencé CI dans les mêmes conditions que celles données dans l'exemple 3. Le test catalytique est également réalisé dans les mêmes conditions que celles de l'exemple 3.

Les performances catalytiques du catalyseur CI sont reportées dans le tableau 7. Il est clair que le catalyseur CI est moins performant que les catalyseurs selon l'invention. En fait ses performances sont proches de celles du catalyseur 2adf défluoré.

EXEMPLE 6 : Catalyseur de comparaison C2

Le catalyseur CI de l'exemple 5 est fluoré par un traitement à 450°C sous une atmosphère contenant CHF3 pendant 6 heures. A l'issue de ce traitement la teneur en fluor du solide est de 0,28 % pds. Le solide ainsi fluoré après la synthèse est mis en forme et testé en aromatisation du pentane dans les mêmes conditions que dans l'exemple 3. Les performances catalytiques du catalyseur C2 ainsi préparé sont reportées dans le tableau 7. Les données de ce tableau 7 indiquent clairement que les performances du catalyseur C2 qui a été fluoré après la synthèse sont inférieures à celles des catalyseurs selon l'invention de teneur en fluor voisine.

TABLEAU 7

PERFORMANCES CATALYTIQUES EN AROMATISATION DU PENTANE
($iC_5$ 60 %, $nC_5$ 40 %) POUR LES SOLIDES PREPARES
DANS LES EXEMPLES 3 A 6.

Les conditions opératoires sont celles de l'exemple n° 3 :
T = 480°C, PPH = 6 $h^{-1}$, P = 1 bar

(performances après 10 cycles réaction - régénération)

| Catalyseur | 1a | 1b | 2a | 2b | 2adf | C1 | C2 |
|---|---|---|---|---|---|---|---|
| % F pds | 0.20 | 0.30 | 0.25 | 0.18 | < 0.02 | 0 | 0.28 |
| PPH ($h^{-1}$) | 6 | 6 | 6 | 6 | 6 | 6 | 6 |
| % conversion | 92 | 87 | 99 | 94 | 86 | 85 | 78 |
| % Sélectivité en aromatiques (pds) | 56 | 53 | 62 | 60 | 50 | 49 | 45 |

**Revendications**

1/ Procédé d'aromatisation d'hydrocarbures renfermant 5 à 7 atomes de carbone par molécule en présence d'un catalyseur du type galloaluminosilicate renfermant en poids :

a) 0,01 à 99,49 % d'une matrice choisie dans le groupe formé par l'alumine, la silice, la magnésie, une argile et toute combinaison d'au moins deux des composés précités et

b) 0,51 à 99,99 % d'une zéolithe synthétisée en milieu fluorure de formule chimique approchée suivante :

$$(M_{(x + y)/n})^{(x + y) +} \quad (Si_{(96-(x + y))} Al_x Ga_y O_{192})^{(x + y)-}$$

M représente un proton et/ou un cation métallique

x est compris entre 0,1 et 9

y est compris entre 0,1 et 7

la zéolithe ayant une teneur en fluor comprise entre 0,02 et 1,5 % poids, le fluor étant incorporé lors de la synthèse, ladite zéolithe étant aussi caractérisée par un diagramme de diffraction X présenté dans les tableaux 1 ou 2.

2/ Procédé selon la revendication 1 dans lequel la zéolithe du dit catalyseur est une zéolithe de type galloaluminosilicate de structure MFI, caractérisée par :

– une teneur en fluor comprise entre 0,02 % et 1,5 % en poids,

– un rapport molaire $si^{IV}/Ga^{III}$ supérieur ou égal à 13,

– un rapport molaire $si^{IV}/Al^{III}$ supérieur ou égal à 10.

**3/** Procédé selon l'une des revendications 1 et 2 dans lequel ladite zéolithe du dit catalyseur, après synthèse en milieu fluorure, a été soumise à un traitement au moins partiel de défluoration permettant d'ajuster ses propriétés acides.

**4/** Procédé selon l'une des revendications 1 à 3, ledit procédé impliquant des régénérations fréquentes du dit catalyseur.

EP 0 472 462 A1

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5 ) |
|---|---|---|---|
| A | EP-A-0 351 312 (INSTITUT FRANCAIS DU PETROLE) --- | | C07C5/41 C07C2/00 C07C15/00 |
| A,D | EP-A-0 351 311 (INSTITUT FRANCAIS DU PETROLE) --- | | |
| A | EP-A-0 252 705 (MOBIL OIL CORPORATION) --- | | |
| A | WO-A-8 910 190 (D. SEDDON) ----- | | |

DOMAINES TECHNIQUES RECHERCHES (Int. Cl.5 )

C07C

Le présent rapport a été établi pour toutes les revendications

| Lieu de la recherche | Date d'achèvement de la recherche | Examinateur |
|---|---|---|
| LA HAYE | 19 NOVEMBRE 1991 | J. VAN GEYT |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons
................................................................
& : membre de la même famille, document correspondant

EPO FORM 1503 03.82 (P0402)

15